# EUROPEAN PATENT SPECIFICATION

(11) **EP 0 587 721 B1**
(45) Date of publication and mention of the grant of the patent: **08.12.1999**
(21) Application number: 92912570.6
(22) Date of filing: 11.05.1992
(51) Int. Cl.: A61F 2/66

(54) **ARTIFICIAL FOOT**
KÜNSTLICHER FUSS
PIED ARTIFICIEL

(30) Priority: 10.05.1991 US 698556; 10.05.1991 US 698557
(43) Date of publication of application: 23.03.1994
(60) Divisional application: 99110279.9
(73) Proprietor: COLLEGE PARK INDUSTRIES, INC., Fraser, MI 48026 (US)
(72) Inventor: SCHEY, Michael, S., Birmingham, MI 48010 (US); ROBINSON, Eric, L., Madison Heights, MI 48071 (US); WOOD, David, B., East Detroit, MI 48021 (US)
(74) Representative: Oppermann, Ewald, Dipl.-Ing.
(86) International application number: US9203931
(87) International publication number: WO9220305

(56) References cited:
- GB-A- 1 371 996
- GB-A- 1 420 627
- GB-A- 2 092 451
- SU-A- 311 635
- US-A- 1 155 545
- US-A- 2 475 372
- US-A- 4 645 509
- US-A- 4 892 554

## Description

### TECHNICAL FIELD

This invention relates to an artificial foot according to the preamble portion of claim 1, and more particularly to a multi-piece prosthetic foot having an ankle member, heel member and toe member.

### Background of the Invention

The basic requirements of an acceptable prosthetic foot are that it will provide a stable support for the amputee throughout a reasonable range of activities and permit the amputee to walk with a normal stride. To achieve this normal stride, the prosthetic foot must mimic the movement of an anatomic foot during walking as the foot continually moves through the heel-strike foot-flat and toe-off cycle. It must also throughout this cycle, provide transverse stability particularly at the toe-off, when the entire weight of the amputee is applied to the forward portion of the prosthetic foot. Prior art prosthetic feet typically are substantially transversely inflexible, which interferes with side to side balancing when walking on uneven surfaces. Unlike a natural foot, the prosthetic foot does not sense or correct itself with this unevenness and an unanticipated sideways tilting of the foot at toe-off results in an imbalance at a critical portion of the stride.

Amputees are no longer satisfied to sit in a wheel chair or be content with a stilted walking motion. An amputee often strives to duplicate physical activities which were conducted before the amputation. These activities may include rigorous physical activities such as running, playing basketball and dancing. Consequently, the artificial foot must also be adaptable to be worn with different styles of shoes.

Prosthetic feet to be commercially acceptable must duplicate the motions of the natural foot as much as possible. These motions include side to side stability at the toe section of the foot where weight can be exerted on each side of the foot. The ankle joint must have torsional flexibility transverse to the up and down motion of the ankle which pivotally lowers and raises the foot.

The added torsional motion of the joint in the artificial foot adds a degree of stress on the resilient pads between the members of the artificial foot not otherwise present in a foot that has limited motion in only the up and down direction relative to pivotable raising and lowering of the foot.

The torsional flexing must be limited within an anatomic range of motion and must be direction sensitive to provide a more lifelike ankle action. Secondly, the resilient pad between the member of the feet must be positively anchored to resist being displaced during torsional motion of the ankle joint.

It is further rehired to have a commercially viable foot that has a formed symmetrical toe member that is used for both left and right feet so that inventory is reduced. The toe member must be shearable to conform to the desired size and to fit within an appropriate left or right shoe.

What is also needed is an ankle member that can be secured to a variety of wedges interposed between the artificial leg and foot such that the artificial foot can be used with high heels or other shoes with varying heel heights.

Document US-A-4 892 554 forming the preamble portion of claim 1 describes a prosthetic foot which includes an ankle member, heel member and an elongate metatarsal-toe member coupled to each other for relative pivotal movement resisted by resilient pads engaged between the respective members. The undersides of the heel and toe members are concavely arched, the length of the arch duplicating that of a natural foot of the same size, with the center of the arch vertically aligned with the center of gravity of the amputee when in a standing, foot-flat position. The toe member is partially bifurcated at its forward end to provide independently flexible toe sections at the inner and outer sides of the foot, achieving a stable, three point support matching that of natural foot.

The object of the present invention is to provide an artificial foot which is capable of more realistically imitating the movement of an anatomic foot during walking.

### Summary of the Invention

The above object is solved by the features indicated in claim 1. Advantageously developed embodiments of the invention are subject-matter of the dependent claims 2 to 11.

According to the invention, the artificial foot comprises a stop limit positioned about the axle joint between the ankle member and the heel member, for providing pivotable motion transverse to the central longitudinal axis of the axle joint along a first direction with respect to the heel member a first predetermined amount, and for providing pivotable motion transverse to the central longitudinal axis along a second direction with respect to the heel member a second different predetermined amount.

In this fashion, a foot having fewer number of inventory parts can be assembled to accommodate high heels and other shoes of different heel heights and provide a torsionally resilient ankle that allows torsional flexing of the foot within limits that are dependent on the relative pivotal position of the heel with respect to the ankle member about the axle joint. Furthermore the bifurcated toe according to claim 8 provides for side to side stability. The symmetrical shape of the formed toe member allows for one toe member to be used for either left or right prosthetic feet. One of the toe members can be cut to allow for confirmation of the small toe side to accommodate smaller sized shoes and feet sizes. In addition, positively anchored elastomeric members are maintained in position in the foot to provide for a stable member against torsional motion of one foot member with respect to another.

### Brief Description of the Drawings

Reference now is made to the accompanying drawings in which:
FIGURE 1 is a perspective view of a prosthetic foot skeletal structure embodying the present invention;
FIGURE 2 is a cross sectional view taken along the lines 2-2 in Figure 1;
FIGURE 3 is a fragmentary cross sectional view taken along the lines 3-3 shown in Figure 2;
FIGURE 4 is a perspective view of the heel member shown in Figure 1;
FIGURE 5 is a top plan view of the toe member shown in Figure 1;
FIGURE 6 is a perspective view of the rear elastomeric pad shown in Figure 1;
FIGURE 7 is a view of the heel toe axle connection partially dissembled for engagement to an assembly and disassembly tool;
FIGURE 8 is a view similar to Figure 7 showing the tool pushing out the axle for disassembly;
FIGURE 9 is a view showing the ankle member mounted to an endoskeleton leg portion
FIGURE 10 is a fragmentary view showing the modified ankle member mounted to an exoskeleton leg portion;
FIGURE 11 is an exploded view illustrating another embodiment of an ankle member and an adjustment wedge;
FIGURE 12 is a fragmentary segmented view showing a prosthetic foot with the ankle and wedge member illustrated in Figure 11 mounted to a prosthetic leg to accommodate high heel shoes; and
FIGURE 13 is a cross sectional view taken along lines 13-13 shown in Figure 12.

### Detailed Description of the Preferred Embodiments

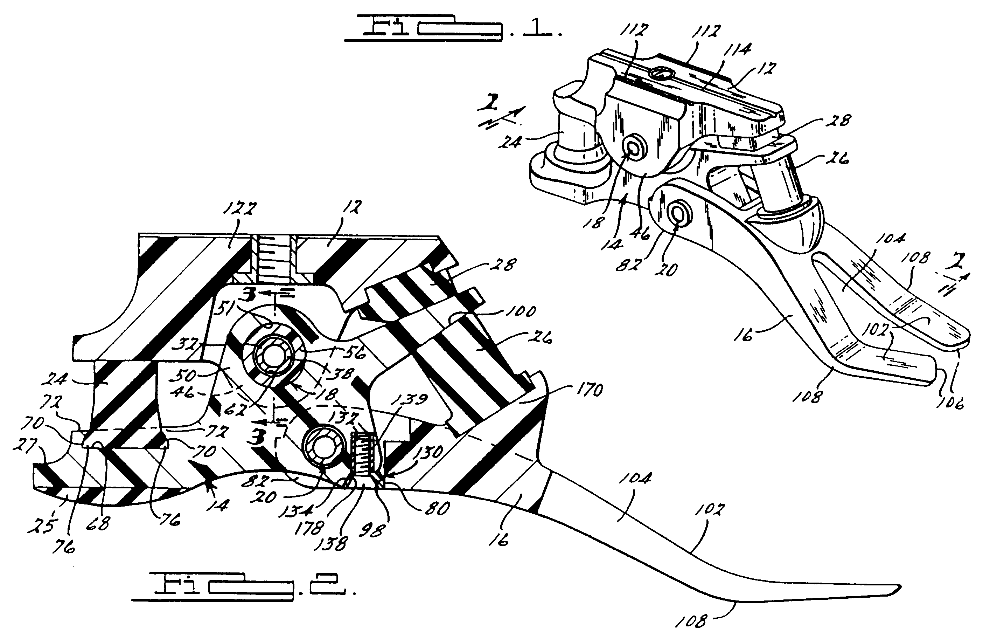

Referring to Figures 1 and 2, a prosthetic foot 10 has an ankle member 12, a heel member 14 and toe member 16. The ankle member 12 is pivotably connected to heel member 14 through axle joint 18. The heel member 14 is pivotably connected to toe member 16 through axle joint 20. The heel, ankle, and toe members 12, 14, 16 are made from a suitable synthetic thermoplastic or composite material such as graphite that can be fiberglass reinforced. The axle joints 18 and 20 provide for pivotable motion of the three members along the axis of the axle joints which runs transverse to the longitudinal axis of the foot; i.e., the length of the foot. Furthermore as explained below, the axle joint 18 provides for limited torsional or pivotable motion about a direction transverse to the longitudinal axis of the axle joint 18.

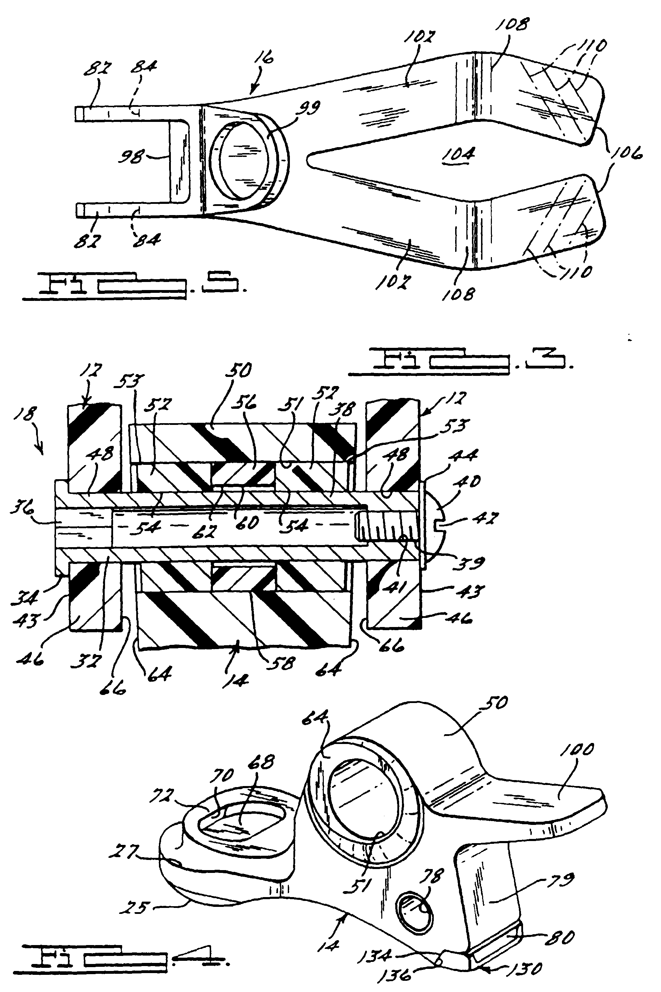

Elastomeric pad 24 provides for resilient resistance of heel member 14 with respect to ankle member 12 against clockwise motion as referenced in Figure 2. Elastomeric pad 26 resists upward counterclockwise motion of toe member 16 with respect to the heel member 14 and elastomeric pad 28 resists clockwise motion of the ankle member 12 with respect to heel member 14. The pads 24, 26, 28 can be selected by size and durometer to accommodate different people's weight, size and personal preferences. A heel pad 25 is bonded to the rear lower section 27 to also absorb shock and vibration. As shown in phantom in Figure 12 when assembled, the elastomeric pad and foot members 12, 14, and 16 can be covered by a firm resilient elastomeric outer layer 30 whose outer surface is cosmetically conformed to that of a natural foot.

Referring now to Figures 2 and 3 the axle joint 18 includes an axle or pin 32 which has one end having an abutment shoulder 34 sized to abut one side 43 of the ankle member 12. The end 35 has a cavity 36 therein which is conformed to non-rotatably receive a tool such as an allen wrench. The pin 32 has a cylindrical section 38 and a second end 39 with an internally threaded section 41 axially positioned within cylindrical section 38. Section 41 receives a threaded fastener 40. The fastener 40 also has a tool receiving cavity or recess 42 therein that can receive an allen wrench or a screwdriver. The fastener 40 may include a washer 44 sized to abut another side 43 of ankle member 12.

The ankle member 12 has two flange sections 46, each with an aperture 48 that receives the pin 32. The flanges 46 are spaced apart to receive the central section 50 of heel member 14. The central section 50 has an aperture 51 which is significantly larger than the outer diameter of pin cylindrical section 38. The pin 32 is integrally formed and extends through both apertures 48 and aperture 51. Because the flange members 46 bear down on the one-piece pin 32 that extends through and beyond aperture 51, a durable and strong axle is formed at axle joint 18. Two rubber or resilient elastomeric bushings 52 have an outer diameter 53 sized to be snugly received in aperture 51 and have an inner diameter 54 sized to snugly receive the cylindrical portion 38 of pin 32. Axially interposed between the bushing 52 is a semi-rigid plastic bushing 56 made from Delrin having an outer diameter 58 sized to be slidably snugly received in aperture 51. The bushing 52 has an inner diameter 60 which is sized larger than the outer diameter of cylindrical section 38 to provide for an annular gap 62 therebetween. The mounting section 50 has tapered sidewalls 64 which are oppositely angled with respect to each other on opposite sides of aperture 50 and are slightly spaced from inner walls 66 of flanges 46.

The bushings 52 provide for both translational motion of the ankle member 12 with respect to the heel member 14 and torsional or pivotal motion which is transverse to the longitudinal axis of pin 32 when in the rest position. The bushings 52 can be selected by durometer to resiliently resist translational and torsional motion of the ankle member 12 with respect to the heel member 14 depending on a person's weight, size and personal preference. However the translational motion is limited by the inner diameter 60 of bushing 56 such that upon translational motion of a predetermined amount, the pin cylindrical section 38 abuts the internal diameter 60 of the bushing 56 which resists further translational motion. Pivoting motion of the pin 32 is limited by the tapered shoulder 64 abutting the interior walls 66 of flanges 46. Because the walls 64 are tapered in a direction as shown in Figure 4, when the ankle member 12 is pivoted either clockwise or counterclockwise from the position shown in Figure 2, the amount of torsional pivoting between the ankle member 12 and heel member 14 will differ. In other words different amounts of torsional motion may be allowed at different relative positions of the ankle member 12 with respect to the heel member 14. Less torsional flexibility may be provided when the elastomeric pad 24 is significantly compressed such that the heel member 14 is rotated clockwise with respect to the ankle member 12. This different torsional motion dependent upon the position of the ankle member 12 more realistically imitates a natural ankle at its varying elevated positions.

Because torsional motion may exert a sideways pulling force on elastomeric pad 24, elastomeric pad 24 needs to be positively engaged within the heel member 14. This is accomplished by a cavity 68 having a recess 70. The heel member 14 has a lip 72 extending over the recess 70 to positively engage the elastomeric pad 24. As shown in Figure 6, the elastomeric pad 24 has a substantially oval lower section 74 with a substantially longer side 75 of the oval section each having a protruding ear 76 which can be received in recess 70.

Referring now to Figures 4, 5, 7 and 8, the axle joint 20 between the heel member 14 and toe member 16 provides pivoting motion of the toe member 16 in an up and down direction. The heel member 14 has a central aperture 78 at a lower section in proximity to an abutment shoulder 80. The toe member 16 has two flanges 82 with apertures 84 therethrough that are aligned with respect to each other. The flanges 82 are spaced apart to receive the lower section 79 of heel member 14. The apertures 78 and 84 are aligned to snugly and slidably receive an integrally formed pin 86. Aperture 78 has two counter bores 81 sized to receive two resilient bushings 83 of desired durometer. The bushings 83 function in a similar fashion to bushings 52 to resiliently resist torsional and translational motion of the toe member 16 relative to the heel member 14. The center section 85 of aperture 78 functions as a limit stop. The center section forms an annular gap 87 therebetween.

Pin 86 is similarly constructed to pin 32 with a flange end 88 having a recess 89 adapted to non-rotatably receive a tool such as an allen wrench. A second end 90 has a threaded cavity 91 adapted to receive either a fastener 40 or a tool 92 as shown in Figures 7 and 8. The pin 86 extends through both flanges 82 of toe member 16 and heel member 14. The tool 92 has a threaded section 93 engagable to threaded section 91 of cylindrical section 94 and hand gripping section 96. A stop shoulder 95 is interposed between the hand gripping section 96 and cylindrical section 94. The stop shoulder 95 can be integral or be a separate clip member 97 engaged in a groove 99. Cylindrical section 94 has an outer diameter equal to the outer diameter of pin 86 that passes through apertures 84 and 78. The assembly of either axle joint 18 or 20 is expedited with the use of tool 92.

The tool as shown in Figure 7 can be engaged with the threaded section 91. The tool is pushed by force exerted on handle section 96 to slide the pin outwardly. The section 92 passes through the apertures 84 and 78 until the stop shoulder 95 abuts the flange 82 as shown in Figure 8. At this time the pin 86 can be unthreaded from section 93. The hand grip then is pulled back such that section 94 is withdrawn from the apertures 84 and 78 to disassemble the axle joint 18. Assembly of the axle joint 18 is achieved by reversing the steps. The pin tool 92 is inserted into the axle apertures. The pin 86 is then threaded onto the section 93 and the handle section 96 is pulled thereby pulling the pin 92 into the apertures. Fastener 40 is then threaded onto pin 32.

A similar process is also used for axle joint 20. The toe section between flanges 82 has an abutment shoulder 98 which abuts the abutment shoulder 80 of heel member 14 to provide for a limit stop in the downward motion of toe member 16 relative to the heel member 14. The toe member 16 has mounting section 170 which receives elastomeric pad 26 which abuts flange section 100 of heel member 14. The elastomeric pad 26 provides resilient resistance against the upward pivotal motion of toe member 16 relative to the heel member 14. The toe member 16 has two symmetrically positioned and mirror image toes 102 with a bifurcating slot 104 therebetween which extends from the front distal ends 106 of toes 102. Each toe 102 has a ball support section 108 which supports weight at the rest position.

The shoulder 80 is on a metal insert member 130 that is fitted in a notch 132 in heel member 14. The member 130 has a rear inclined surface 134 that abuts inclined surface 136 of heel member 14. The member 130 has an aperture 178 therethrough that receives screw 138 that engages threads 139 in heel member 14.

After the pin 86 is installed in ankle joint 20, the pad 26 can be preloaded by installing the metal insert member 130. As the screw 138 is tightened, the inclined surfaces 134 and 136 force the member 130 forward to abut shoulder 98 and thereby pivot the toe member 16 in a counter-clockwise direction as shown in Figure 2 which places a preload on pad 26.

The preloading of pad 26 improves its wear and durability. The elastomeric pad 26 is preferably compressed to its compression set point. When such preloading of pad 26 occurs, the toe member 16 remains loaded against insert member 130 and any undesirable loosening of the toe member 16 with respect to the heel member 14 is eliminated. Readjustment of the toe member 16 with the heel member 14 becomes unnecessary. The preloading also changes the feel of the foot. The preloaded pad 26 feels like a larger pad that is not under a preload. As such a stiffer foot is possible without increasing the size of the components.

The metal insert member 130 also allows repair or replacement of elastomeric pad 26 without the need to disassemble axle joint 20. When the insert member 130 may be disassembled from heel member 14, toe member 16 is then free to pivot clockwise as shown in Figure 2 displacing pad 26 to an accessible position. The pad 26 now spaced from flange section 100 may be repaired or replaced.

As shown in Figure 5, the symmetry of the toes 102 allows for one toe member 16 to be manufactured for both left and right feet. If a particular foot size is small, for contour purposes the outer or small toes need to be shortened. This contour can be easily accomplished by cutting one of the toes 102 for example along one of the dotted lines 110 to provide for a shorter outer toe which would fit within a smaller cosmetic outer foot cover and a smaller sized shoe. However for standard and larger feet, the toes 102 need no trimming. The ball or weight bearing sections 108 are independently flexible such that one toe 102 can be flexed and change its height relative to the vertical position of the other toe 102.

The ankle member 12 is adaptable to be attached to either an endoskeleton as shown in Figure 9 or an exoskeleton as shown in Figure 10. The ankle member has longitudinal slots 112 and 114 which non-rotatably engage standard attachments such as Otto Bock endoskeletal and exoskeletal systems and/or adapters. The endoskeletal and exoskeletal systems as shown in Figures 9 and 10 are standard and form no part of the invention. The ankle member 12, however, is adaptable to be connected to either system. The ankle member 12 can be connected to an exoskeletal connection 118 as shown in Figure 10. A bolt 174 having an enlarged head 176 passes upward through the aperture 121 with the bolt head engaging the recessed flange 131 of ankle member 14. The threaded portion 133 of the bolt then engages the endoskeleton fastener 135.

For attachment to an endoskeleton as shown in Figure 9, a top portion 122 of the ankle 12 has the aperture 120 resized. Threaded insert 118 self taps in aperture 120. The threaded insert 172 has an interior opposite threaded aperture 124. A bolt 127 extends through the endoskeletal adaptor 126 and threadably engages threaded aperture 124.

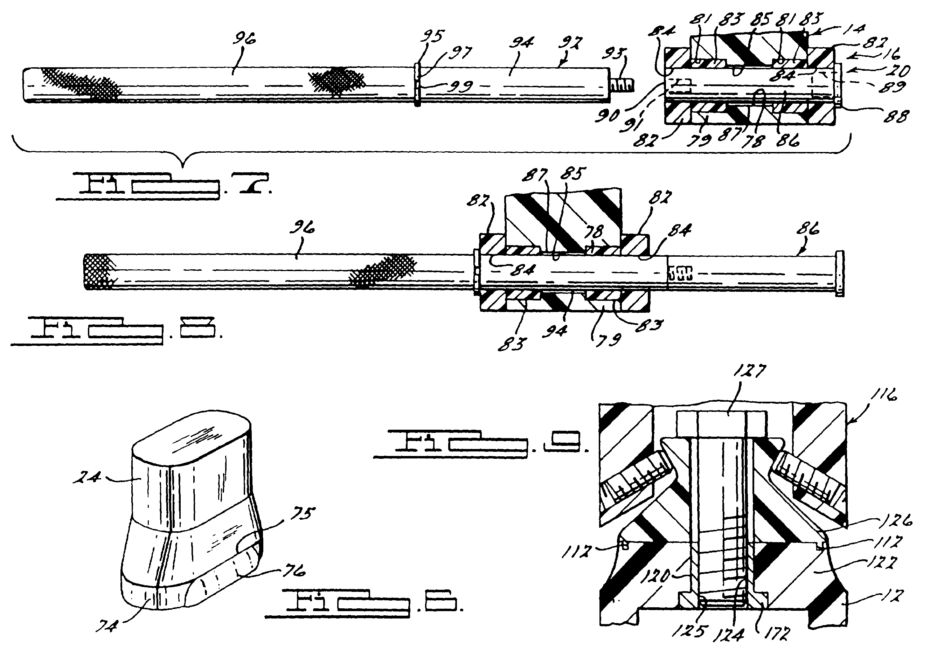

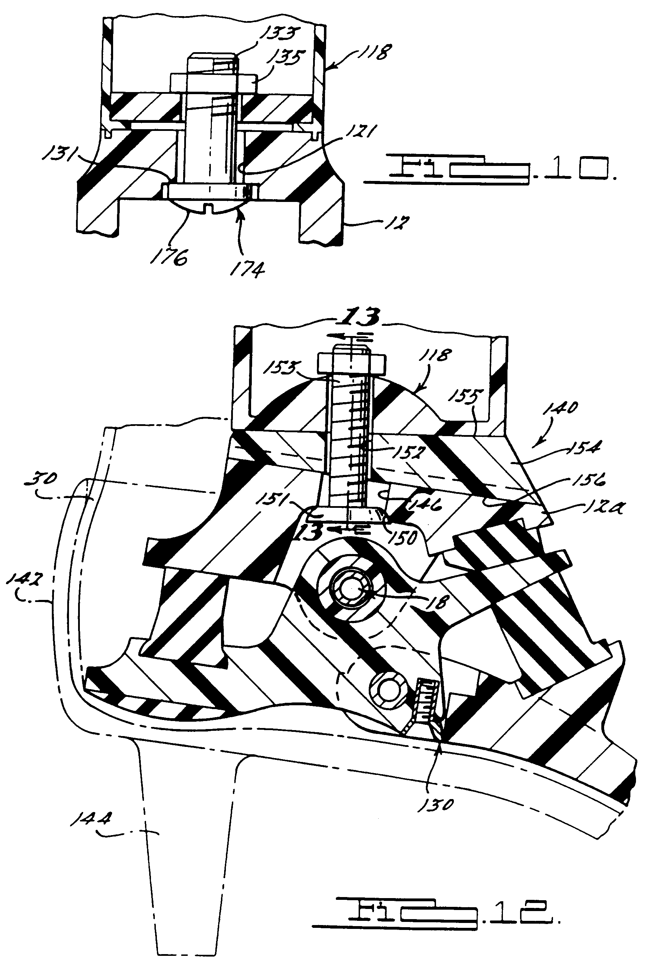

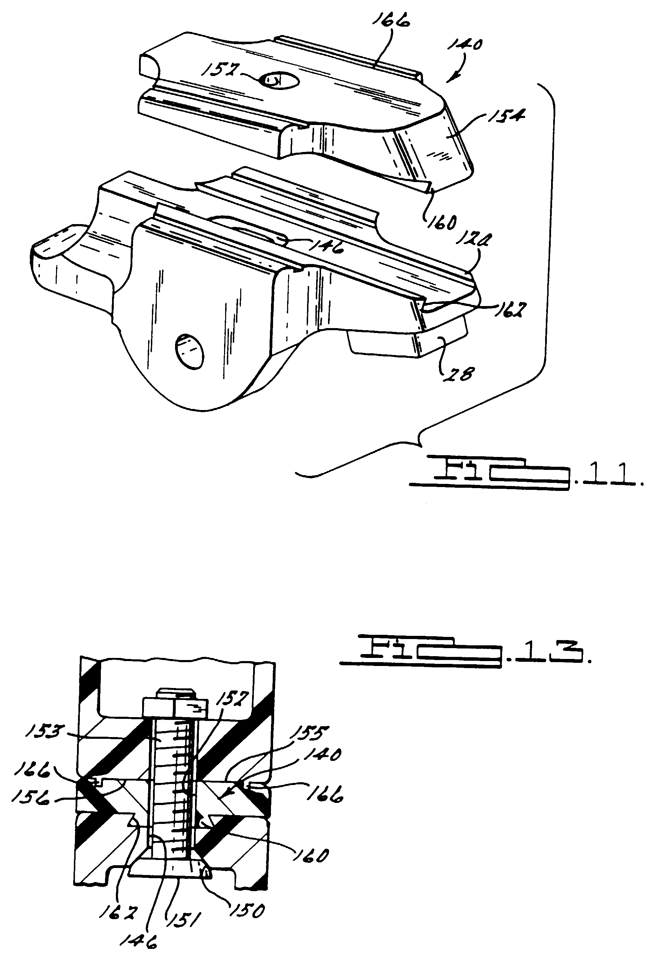

Referring to Figures 11, 12 and 13, the prosthetic foot 10 has a modified member 12a connected to an adjustment attachment mechanism 140 to compensate for shoes 142 having high heels 144 or different heel heights. As shown in Figure 11, the lower surface 156 of member 154 has a dove tail 160 to engage a dove tail slot 162 longitudinally extending along the upper surface 164 of the ankle member 12a. The ankle member 12a has a slot 146 with a contoured seat section 150. The seat section 150 engages a semi spherically contoured bolt head 151. The bolt 153 passes through the slot 146 and through an aperture 152 of a wedge member 154 to engage attachment mechanism 118 of exoskeleton shown in Figure 10. The aperture 152 has its axis perpendicular to its upper surface 155 and that the wedge member 154 is fixed positioned on ankle member 12a. The upper surface 155 may have longitudinal 166 grooves to non-rotatably engage the leg member. The foot when at the rest position is downwardly extended relative to the foot shown in Figure 2. Different wedge member 154 having different degrees of angles between the surfaces 155 and 156 can accommodate for different shoe heel heights. The slot 146 provides for the bolt 153 passing through the ankle member 12a at different angles. The seat 150 is spherical in shape to provide for flush seating of the complementary semi-spherical shaped bolt head 151 at the various angles. The dove tail slot 162 and dove tail 160 prevent the ankle member 12a and wedge member 154 from relative rotation. The ankle joint 18 is aligned vertically below the bolt 153 and slot 146.

Variations and modifications of the present invention ar possible without departing from its scope as defined by the appended claims.

## Claims

1. An artificial foot, comprising
an ankle member (12) and a heel member (14) with said heel member (14) pivotally connected to said ankle member (12) for resiliently resisted pivotal motion about a transversely extending axle joint (18) having a central longitudinal axis,
said heel member (14) having at least one transverse aperture (51) aligned with at least one aperture (48) in said ankle member (12) to form a passage through said heel and ankle members (12, 14), through which an axle pin member (32) passes,
said axle pin member (32) being surrounded by at least one resilient bushing (52) accommodating limited resiliently resisted movement transverse to the longitudinal axis of said axle pin member (32) of said heel member (14) relative to said ankle member (12),
**characterized by** a stop limit (56, 64, 66) positioned about said axle joint (18) for providing said pivotable motion transverse to said central longitudinal axis along a first direction with respect to said heel member (14) a first predetermined amount and for providing pivotable motion transverse to said central longitudinal axis along a second direction with respect to said heel member (14) a second different predetermined amount.

2. An artificial foot according to claim 1, wherein said stop limit (56, 64, 66) includes first and second oppositely canted abutments (64, 66) on one of said heel and ankle members (12, 14) positioned about said axle joint (18) for abutment with complementary abutments (64, 66) on said other of said heel and ankle members (12, 14).

3. An artificial foot according to claim 2, wherein said canted abutments (64, 66) are formed by annular flat shoulders canted with regard to the first transverse axis.

4. An artificial foot according to claim 1, wherein
at least one resilient elastomeric pad (24) is interposed between said heel member (14) and one of said ankle and toe members (12, 16) for resiliently resisting relative pivotable motion of said heel member (14) in one direction about said axle joint (18),
said elastomeric pad (24) having one end mounted in a recess (70) in one (14) of said members (12, 14, 16),
said recess (70) having an undercut and said one member (14) having a lip portion (72) over said undercut to positively engage said elastomeric pad (24) to prevent said elastomeric pad (24) from being pulled out from said recess (70) due to torsional stresses exerted by the limited transverse pivotal motion of said members (12, 14, 16),
said one end of said elastomeric pad (24) being substantially oval in shape with long sides (75) of said oval having outwardly extending ears (76) for engagement with said recess (70).

5. An artificial foot according to claim 1, wherein
one (14) of said heel and ankle members (12, 14) has one central aperture (51) transversely passing therethrough,
the other (12) of said heel and ankle members (12, 14) having two shoulder sections (46) with an aperture (48) through each shoulder section (46) aligned with each other,
said one member (14) being positionable between said shoulder sections (46) of said other member (12),
said central aperture (51) having a diameter larger than the diameter of said axle pin member (32),
said central aperture (51) having a pair of resilient bushings (52) positioned within said central aperture (51) near respective ends thereof, each bushing (52) having an inner diameter sized to snugly receive said axle pin member (32), and wherein
a stop limit (56) is positioned between said resilient bushings (52) for limiting motion for said axle pin member (32) transverse to its longitudinal axis.

6. An artificial foot according to claim 5, wherein said stop limit (56) is a semi-rigid bushing fitted in said central aperture (51) having an outer diameter substantially equal to the diameter of said central aperture (51) and an inner diameter (60) slightly larger than said diameter of said axle pin member (32).

7. An artificial foot according to claim 5, wherein said one member (14) with said central aperture (51) has tapered shoulders (64) to provide a predetermined limit of pivotable motion along a first direction and a second predetermined limit of pivotable motion along a second direction between said heel and ankle members (12, 14) with said pivotable motion being transverse to said longitudinal axis of said axle pin member (32).

8. An artificial foot according to claim 1, further comprising a toe region (16) having an elongated slot (104) therethrough extending longitudinally rearwardly from a forward end of said toe region (16) and bifurcating said toe region (16) into two symmetrically positioned and substantially mirror imaged toe sections (102).

9. An artificial foot according to claim 8, wherein
said toe region includes a separate toe member, and further comprising
a pivotable connection (20) between said heel member (14) and said toe member (16) for resiliently resisted pivotal movement about a second transverse axis,
said heel member (14) and said toe member (16) each having a central abutment shoulder (80, 98) that abut each other to define an end limit to downward pivotable movement of a forward section of said toe member (16) with respect to said heel member (14).

10. An artificial foot according to claim 1, wherein
said ankle member (12) has an aperture (120) therethrough at an upper section that receives a bolt (127) for connection to an exoskeletal leg member and a threaded insert (118) insertable in said aperture (120) for connection to an adapter (126) and a threaded fastener extending downward from said endoskeletal leg member through said adapter (126),
said ankle member (12) and said adapter (126) being nonrotatably connected to each other.

11. An artificial foot according to claim 8, wherein said toe sections (102) diverge forwardly from a pivotable connection (20) of said toe region (16) with said heel member (14) to a weight supporting section (108) at each toe section (102).

## Patentansprüche

1. Künstlicher Fuß mit
einem Knöchelteil (12) und einem Fersenteil (14), wobei das Fersenteil (14) für eine Schwenkbewegung gegen einen elastischen Widerstand um ein sich in Querrichtung erstreckendes Achsgelenk (18), welches eine zentrale Längsachse hat, schwenkbar mit dem Knöchelteil (12) verbunden ist,
wobei das Fersenteil (14) mindestens eine Queröffnung (51) aufweist, welche mit mindestens einer Öffnung (48) in dem Knöchelteil (12) ausgefluchtet ist, um einen Durchgang durch die Fersen- und Knöchelteile (12, 14) auszubilden, durch welchen ein Achszapfenteil (32) hindurchläuft,
wobei das Achszapfenteil (32) durch mindestens ein elastisches Lager (52) umgeben ist, das eine begrenzte Bewegung des Fersenteils (14) relativ zu dem Knöchelteil (12) gegen einen elastischen Widerstand quer zu der Längsachse des Achszapfenteils (32) aufnimmt,
**gekennzeichnet durch** eine Anschlagbegrenzung (56, 64, 66), die um das Achsgelenk (18) herum angeordnet ist, derart, daß sie die Schwenkbewegung quer zu der zentralen Längsachse entlang einer ersten Richtung bezüglich des Fersenteils (14) um einen ersten vorbestimmten Betrag gestattet und eine Schwenkbewegung quer zu der zentralen Längsachse entlang einer zweiten Richtung bezüglich des Fersenteils (14) um einen zweiten verschiedenen vorbestimmten Betrag gestattet.

2. Künstlicher Fuß nach Anspruch 1, wobei die Anschlagbegrenzung (56, 64, 66) erste und zweite entgegengesetzt abgeschrägte Widerlager (64, 66) an einem der Fersen- und Knöchelteile (12, 14) umfaßt, die für eine Anlage an komplementären Widerlagern (64, 66) an dem anderen der Fersen- und Knöchelteile (12, 14) um das Achsgelenk (18) herum angeordnet sind.

3. Künstlicher Fuß nach Anspruch 2, wobei die abgeschrägten Widerlager (64, 66) durch ringförmige flache Schultern ausgebildet sind, die bezüglich der ersten Querachse abgeschrägt sind.

4. Künstlicher Fuß nach Anspruch 1, wobei
mindestens ein elastischer gummiartiger Puffer (24) zwischen dem Fersenteil (14) und einem der Knöchel- und Zehenteile (12, 16) zwischengefügt ist, um einer relativen Schwenkbewegung des Fersenteils (14) in einer Richtung um das Achsgelenk (18) einen elastischen Widerstand entgegenzusetzen,
wobei der gummiartige Puffer (24) ein Ende hat, welches in einer Aussparung (70) in einem (14) der Teile (12, 14, 16) befestigt ist,
wobei die Aussparung (70) einen Hinterschnitt hat und das eine Teil (14) einen Lippenabschnitt (72) aufweist, der über dem Hinterschnitt liegt, um mit dem gummiartigen Puffer (24) formschlüssig einzugreifen, um den gummiartigen Puffer (24) daran zu hindern, aus der Aussparung (70) aufgrund von Torsionsspannungen, die durch die begrenzte Schwenkbewegung der Teile (12, 14, 16) in Querrichtung aufgebracht werden, herausgezogen zu werden,
wobei das eine Ende des gummiartigen Puffers (24) im wesentlichen eine ovale Form hat, wobei lange Seiten (75) des Ovals sich nach außen erstreckende Ansätze (76) zum Eingriff mit der Aussparung (70) aufweisen.

5. Künstlicher Fuß nach Anspruch 1, wobei
eines (14) der Fersen- und Knöchelteile (12, 14) eine zentrale Öffnung (51) hat, die dort in Querrichtung hindurch verläuft,
wobei das andere (12) der Fersen- und Knöchelteile (12, 14) zwei Schulterabschnitte (46) aufweist, mit einer Öffnung (48) durch jeden Schulterabschnitt (46), die miteinander ausgefluchtet sind,
wobei das eine Teil (14) zwischen den Schulterabschnitten (46) des anderen Teils (12) angeordnet werden kann,
wobei die zentrale Öffnung (51) einen Durchmesser aufweist, der größer ist als der Durchmesser des Achszapfenteils (32),
wobei die zentrale Öffnung (51) ein Paar von elastischen Lagern (52) hat, die in der zentralen Öffnung (51) in der Nähe ihrer jeweiligen Enden angeordnet sind, wobei jedes Lager (52) einen Innendurchmesser hat, der dimensioniert ist, um das Achszapfenteil (32) eng aufzunehmen, und wobei
eine Anschlagbegrenzung (56) zwischen den elastischen Lagern (52) angeordnet ist, um die Bewegung für das Achszapfenteil (32) quer zu seiner Längsachse zu begrenzen.

6. Künstlicher Fuß nach Anspruch 5, wobei die Anschlagbegrenzung (56) ein halbstarres Lager ist, das in der zentralen Öffnung (51) eingepaßt ist und einen Außendurchmesser hat, der im wesentlichen gleich dem Durchmesser der zentralen Öffnung (51) ist sowie einen Innendurchmesser (60) aufweist, der geringfügig größer als der Durchmesser des Achszapfenteils (32) ist.

7. Künstlicher Fuß nach Anspruch 5, wobei das eine Teil (14) mit der zentralen Öffnung (51) abgeschrägte Schultern (64) aufweist, um eine vorbestimmte Begrenzung der Schwenkbewegung entlang einer ersten Richtung und eine zweite vorbestimmte Begrenzung der Schwenkbewegung entlang einer zweiten Richtung zwischen den Fersen- und Knöchelteilen (12, 14) bereitzustellen, wobei die Schwenkbewegung quer zu der Längsachse des Achszapfenteils (32) verläuft.

8. Künstlicher Fuß nach Anspruch 1, weiterhin umfassend einen Zehenbereich (16), der durch sich hindurch einen langgestreckten Schlitz (104) aufweist, welcher sich in Längsrichtung nach hinten von einem vorderen Ende des Zehenbereichs (16) erstreckt und den Zehenbereich (16) in zwei symmetrisch angeordnete und im wesentlichen spiegelbildliche Zehenabschnitte (102) gabelt.

9. Künstlicher Fuß nach Anspruch 8, wobei
der Zehenbereich ein separates Zehenteil aufweist, und ferner umfassend
eine Schwenkverbindung (20) zwischen dem Fersenteil (14) und dem Zehenteil (16) für eine Schwenkbewegung um eine zweite Querachse gegen einen elastischen Widerstand,
wobei das Fersenteil (14) und das Zehenteil (16) jeweils eine zentrale Widerlagerschulter (80, 98) aufweisen, die einander anliegen, um eine Endbegrenzung für eine nach unten gerichtete Schwenkbewegung eines vorderen Abschnitts des Zehenteils (16) bezüglich des Fersenteils (14) auszubilden.

10. Künstlicher Fuß nach Anspruch 1, wobei
das Knöchelteil (12) an einem oberen Abschnitt eine Öffnung (120) dort hindurch hat, welche einen Bolzen (127) zur Verbindung mit einem exoskelettalen Beinteil aufnimmt, und einen Gewindeeinsatz (118) aufweist, der in die Öffnung (120) zur Verbindung mit einem Adapter (126) einsetzbar ist, wobei sich ein mit einem Gewinde versehenes Befestigungsmittel von einem endoskelettalen Beinteil nach unten durch den Adapter (126) hindurch erstreckt,
wobei das Knöchelteil (12) und der Adapter (126) unverdrehbar miteinander verbunden sind.

11. Künstlicher Fuß nach Anspruch 8, wobei die Zehenabschnitte (102) von einer Schwenkverbindung (20) des Zehenbereichs (16) mit dem Fersenteil (14) zu einem Gewichtsauflagerabschnitt (108) an jedem Zehenabschnitt (102) nach vorn auseinanderlaufen.

## Revendications

1. Pied artificiel comprenant
un élément formant cheville (12) et un élément formant talon (14), ledit élément formant talon (14) étant relié de manière pivotante audit élément formant cheville (12) en vue d'un mouvement de pivotement soumis à une résistance élastique autour d'un joint d'articulation (18) s'étendant transversalement et comportant un axe longitudinal central,
ledit élément formant talon (14) comportant au moins un trou transversal (51) en alignement avec au moins un trou (48) prévu dans ledit élément formant cheville (12) pour former à travers lesdits éléments formant talon et cheville (12, 14) un passage à travers lequel passe un élément formant axe d'articulation (32),
ledit élément formant axe d'articulation (32) étant entouré par au moins un fourreau élastique (52) permettant un mouvement limité soumis à une résistance élastique, transversalement à l'axe longitudinal dudit élément formant axe d'articulation (32), dudit élément formant talon (14) par rapport audit élément formant cheville (12),
**caractérisé par** une limite d'arrêt (56, 64, 66) positionnée autour dudit joint d'articulation (18) pour permettre ledit mouvement de pivotement transversalement audit axe longitudinal central suivant une première direction par rapport audit élément formant talon (14), avec une première amplitude prédéterminée, et un mouvement de pivotement transversalement audit axe longitudinal central suivant une seconde direction par rapport audit élément formant talon (14), avec une seconde amplitude prédéterminée différente.

2. Pied artificiel selon la revendication 1, dans lequel ladite limite d'arrêt (56, 64, 66) comprend, sur l'un desdits éléments formant talon et cheville (12, 14), des premier et second éléments de butée inclinés (64, 66) positionnés autour dudit joint d'articulation (18) pour venir en butée contre des éléments de butée (64, 66) complémentaires prévus sur l'autre desdits éléments formant talon et cheville (12, 14).

3. Pied artificiel selon la revendication 2, dans lequel lesdits éléments de butée inclinés (64, 66) sont formés par des épaulements plats annulaires inclinés par rapport au premier axe transversal.

4. Pied artificiel selon la revendication 1, dans lequel
au moins un coussinet en élastomère élastique (24) est interposé entre ledit élément formant talon (14) et l'un desdits éléments formant cheville et orteils (12, 16) pour résister élastiquement à un mouvement de pivotement relatif dudit élément formant talon (14) dans une seule direction autour dudit joint d'articulation (18),
ledit coussinet en élastomère (24) ayant une extrémité montée dans une cavité (70) prévue dans l'un (14) desdits éléments (12, 14, 16),
ladite cavité (70) comportant un renfoncement et ledit élément (14) comportant une partie formant lèvre (72) qui recouvre ledit renfoncement pour venir efficacement en prise avec ledit coussinet en élastomère (24) afin d'empêcher que ce dernier ne soit extrait par traction de ladite cavité (70) sous l'effet de contraintes de torsion exercées par le mouvement de pivotement transversal limité desdits éléments (12, 14, 16),
ladite extrémité dudit coussinet en élastomère (24) ayant une configuration sensiblement ovale dont les grands côtés (75) comporte des oreilles (76) s'étendant vers l'extérieur pour venir en prise avec ladite cavité (70).

5. Pied artificiel selon la revendication 1, dans lequel
l'un (14) desdits éléments formant talon et cheville (12, 14) comporte un trou central (51) qui le traverse transversalement, tandis que
l'autre (12) desdits éléments formant talon et cheville (12, 14) comporte deux parties formant épaulements (46) respectivement traversées par des trous (48) en alignement l'un avec l'autre,
ledit premier élément (14) pouvant être positionné entre lesdites parties formant épaulements (46) dudit second élément (12),
ledit trou central (51) ayant un diamètre supérieur à celui dudit élément formant axe d'articulation (32),
ledit trou central (51) comportant deux fourreaux élastiques (52) positionnés à l'intérieur de lui à proximité de ses extrémités respectives, chaque fourreau (52) ayant un diamètre intérieur dimensionné pour recevoir par un ajustement à frottement doux ledit élément formant axe d'articulation (32), et dans lequel
une limite d'arrêt (56) est positionnée entre lesdits fourreaux élastiques (52) pour limiter un déplacement dudit élément formant axe d'articulation (32) transversalement à son axe longitudinal.

6. Pied artificiel selon la revendication 5, dans lequel ladite limite d'arrêt (56) est un fourreau semi-rigide inséré dans ledit trou central (51), qui a un diamètre extérieur sensiblement égal à celui dudit trou central (51) et un diamètre intérieur (60) légèrement supérieur à celui dudit élément formant axe d'articulation (32).

7. Pied artificiel selon la revendication 5, dans lequel ledit premier élément (14) muni dudit trou central (51) comporte des épaulements obliques (64) pour définir une première limite prédéterminée de mouvement de pivotement suivant une première direction et une seconde limite prédéterminée de mouvement de pivotement suivant une seconde direction entre lesdits éléments formant talon et cheville (12, 14), ledit mouvement de pivotement étant transversal audit axe longitudinal dudit élément formant axe de pivotement (32).

8. Pied artificiel selon la revendication 1, comprenant également une zone d'orteils (16) à travers laquelle une fente allongée (104) s'étend longitudinalement vers l'arrière depuis une extrémité avant de ladite zone d'orteils (16) et divise cette dernière en deux parties formant orteils (102) positionnées de manière symétrique et sensiblement énantiomorphes.

9. Pied artificiel selon la revendication 8, dans lequel ladite zone d'orteils comprend un élément formant orteils séparé, et également
une liaison pivotante (20) entre ledit élément formant talon (14) et ledit élément formant orteils (16) pour permettre un mouvement de pivotement soumis à une résistance élastique autour d'un second axe transversal,
ledit élément formant talon (14) et ledit élément formant orteils (16) comportant respectivement des épaulements de butée centraux (80, 98) qui viennent en butée l'un contre l'autre afin de définir une limite extrême pour un mouvement de pivotement vers le bas de ladite partie avant dudit élément formant orteils (16) par rapport audit élément formant talon (14).

10. Pied artificiel selon la revendication 1, dans lequel
ledit élément formant cheville (12) comporte un trou (120) qui le traverse au niveau d'une partie supérieure et qui reçoit un boulon (127) en vue d'une liaison avec un élément formant jambe exosquelettique, et une pièce d'insertion filetée (118) apte à être insérée dans ledit trou (120) en vue d'une liaison avec un adaptateur (126) et un organe de fixation fileté qui s'étend vers le bas depuis ledit élément formant jambe endosquelettique à travers ledit adaptateur (126),
ledit élément formant cheville (12) et ledit adaptateur (126) étant reliés l'un à l'autre de manière non rotative.

11. Pied artificiel selon la revendication 8, dans lequel lesdites parties formant orteils (102) divergent vers l'avant depuis une liaison pivotante (20) de ladite zone d'orteils (16) avec ledit élément formant talon (14) jusqu'à une partie porteuse de poids (108) au niveau de chaque partie formant orteil (102).
